# EUROPEAN PATENT SPECIFICATION

(11) **EP 3 381 418 B1**
(45) Date of publication and mention of the grant of the patent: **29.01.2020**
(21) Application number: 16868068.4
(22) Date of filing: 23.11.2016
(51) Int. Cl.: A61F 5/58, A61G 1/04, A61F 5/058, A61G 1/044

(54) **SPECIFIC IMMOBILISATION DEVICE**
SPEZIFISCHE IMMOBILISIERUNGSVORRICHTUNG
DISPOSITIF D'IMMOBILISATION SPÉCIFIQUE (DIE)

(30) Priority: 25.11.2015 ES 201500885; 18.10.2016 ES 201600913
(43) Date of publication of application: 03.10.2018
(73) Proprietor: Sapíña Silvestre, Miguel Angel, 04001 Almería (ES)
(72) Inventor: Sapíña Silvestre, Miguel Angel, 04001 Almería (ES)
(74) Representative: Ibarra Garcia, Isabel
(86) International application number: PCT/ES2016/000130
(87) International publication number: WO 2017/089629

(56) References cited:
- WO-A1-01/37764
- CN-Y- 200 970 289
- CN-Y- 201 308 562
- GB-A- 2 156 226
- GB-A- 2 507 157
- US-A- 4 580 555
- US-A- 5 101 815
- US-A1- 2006 184 083

## Description

### TECHNICAL FIELD

The present device belongs to the field of medical-health equipment, and more specifically to the field of pre-hospital medical emergency and patient transport equipment and devices. The device constitutes a "specific immobilisation device" (or its abbreviation, SID) which allows a perfect "en bloc" immobilisation of the site of injury and the affected lower limb in cases of femoral neck fracture, and the main purpose of which is to enable an efficacious, efficient, and effective rescue of the injured subject, all without requiring the administration of medication to the patient, and accordingly without requiring the intervention of a medical professional during the rescue process. This device can also be used with the same efficacy, efficiency, and effectiveness as a specific immobilisation device (SID) in cases of pelvic fracture, femoral fracture, knee fracture, and fracture from the proximal tibia to the knee.

### BACKGROUND AND CURRENT STATE OF THE ART

The idea to create this device comes from the need observed by a professional in his day-to-day activity as part of an out-of-hospital medical emergency team, who finds that current rescue teams do not have any specific immobilisation device for femoral neck fractures which allows evacuating an injured patient with his injured limb completely and properly immobilised.

GB2507157A discloses a medical device for hip support of a patient. The document includes attachment means for securing the device to the patient, a hinge assembly comprising first and second section pivotally connected to each other.

For descriptive purposes, "rescue process" (understood as a whole, i.e., from the moment the emergency team reaches the scene of the accident in response to a distress call until the moment the patient is brought to a traumatologist in an emergency department in the hospital area) shall herein be understood as being made up of three phases: (i) a first phase of evacuating the patient from the place where he lies to the rescue vehicle, (ii) a second phase consisting of transferring the patient in the corresponding rescue vehicle, and (iii) a third phase consisting of hospital staff receiving the patient in the hospital.

At present, a professional rescuer reaches the scene of the accident and performs a first evaluation of the injured subject to establish the extent of his injury/injuries. At present, in cases in which the patient is suspected of having a femoral neck fracture injury, emergency professionals will proceed to get (without using any specific device) the injured subject off the ground with a "scoop stretcher" and keep him still on the stretcher with the fastening straps that are provided. The subject is evacuated without any type of specific immobilisation and is only "properly immobilised" upon reaching the emergency vehicle when he is placed on a so-called "vacuum mattress" (a general immobiliser designed for use with polytraumatism patients) that has been previously spread over the stretcher of the rescue vehicle or ambulance.

The use of said "vacuum mattress" as an immobiliser in case of such a specific and localised fracture has the drawback of making the patient feel uncomfortable and overwhelmed while he is being transported to the hospital, given that the mattress immobilises the entire body of the patient, not allowing any part of his body to move. Furthermore, the current lack of specific immobilisation in cases of femoral neck fracture exacerbates the pain experienced by the injured subject during his evacuation from the place where he lies to the rescue vehicle. Said lack of specific immobilisation in this first rescue phase (and the subsequent exacerbation of the pain that takes place) makes it necessary to administer medication (morphine derivatives, pain killers) in order to make the pain the patient experiences during said process more bearable. The use of said immobilisation technique today, used exclusively during the trip for transferring the patient in the vehicle to the hospital, not only makes it necessary, as mentioned, for the evacuation from where the patient lies to the rescue vehicle to be carried out without any type of specific immobilisation, but also makes it necessary to deprive the patient, upon his arrival to the hospital, of the immobilisation provided to him by the "vacuum mattress" so that he can be transferred to a hospital stretcher, where the injured limb of the patient will once again be without any type of specific immobilisation during the first hospital stage, i.e., reception, evaluation, and x-rays.

In cities, the evacuation of the patient from inside offices to the ambulance usually involves the added difficulty of having to carry the patient down to the street from a certain floor. This entails putting the patient in a tilted position (if the patient is carried down using the stairs) or even a vertical position (if the patient is carried down using the lift). Placing the patient in said position without a specific immobilisation (bearing in mind that the general immobilisation, i.e., the "vacuum mattress", is not put in place until the patient reaches the stretcher of the emergency vehicle) causes a great deal of pain that the patient must withstand and cope with however he can.

Precisely said lack of specific immobilisation leads to the intervention of a physician being imperative in these cases; in other words, in Spain, an entire advanced life support (ALS) team [which, in Spain, is made up of a physician, a nurse, and a emergency medical technician] must go to the scene of the accident so that they can treat the injured patient to ease his pain. In Spain, medication is an advanced life support technique that the two emergency medical technicians (EMTs) of a basic life support (BLS) team is not authorized to perform.

Accordingly, despite the amount of time [transferring to/from the vacuum mattress] and resources [physician/medication] invested today in rescuing an injured subject with femoral neck fracture, pain is still very much present during evacuation, given that the patient is conscious and the medication (morphine derivative), administered by protocol, reduces but does not eliminate pain that is exacerbated by the handling and movements typical of a rescue of this type. The foregoing may apply to those cases in which the injured patient lies in a place that is hard, difficult, or impossible for the rescue vehicle to access, such as, for example, at sea, in the countryside, on a mountain, etc; or in those cases in which a physician is not available or cannot reach the injured subject on time, with professional rescue staff who are not physicians going to the scene.

The device that will be described below is therefore defined as a specific immobilisation device (SID) for cases of femoral neck fracture, an injury for which the pre-hospital emergency staff in Spain are not currently using any type of specific immobilisation device (SID).

### DISCLOSURE OF THE DEVICE

The invention is defined by the appended claims.

The design of the device allows the rescue staff to carry out rescue operations, maximally reducing the pain experienced by the injured subject as a result of the handling and movements typical of the evacuation, the transfer to the hospital centre, and the reception of the patient by hospital staff.

The specific immobilisation device has the following outstanding features:
Anatomical shape: the anatomical shape with which the specific immobilisation device has been designed allows immobilising the patient efficiently, where it perfectly adapts to the anatomy of persons of any type, as will be seen below.

Central guide having a telescopic structure: the guide allows aligning the affected lower limb when there is a fracture, adapting its length to the physical characteristics (leg length) of the injured subject, and accordingly allowing the "en bloc" immobilisation of the affected extremity, completing a perfect immobilisation of the affected limb from the pelvis to the ankle.

Central guide with traction function: the central guide allows the limb to go back to its original position and to remain in said situation throughout the entire process of rescuing, evacuating, and transporting the patient to the hospital. The purpose of traction is to minimize the pain suffered by the patient due to the fracture (femoral fracture or fracture from the proximal tibia to the knee, and in some cases femoral neck fracture) so that the patient can be rescued without having to administer any medication. Traction will be performed forward or backward according to the type and characteristics of the injury suffered by the subject, preventing the injury from worsening.

Materials used for the outer cover: (i) non-slip materials: the material covering the guide is a non-slip material, furthermore having small, strategically placed, silicone ovals or ovals which are made of another material having similar characteristics or properties to reinforce said function. In this manner, the patient is prevented from painfully sliding down when he is placed in a tilted or vertical position during evacuation; (ii) hygienic materials that are easy to clean and maintain: the material covering the outside of the immobilisation device is easy and fast to clean, where it can be quickly put in use again after a prior intervention that has ended; impermeable materials: the material covering the outside of the device is impermeable so that it can be used in any condition and under any circumstance, particularly regardless of adverse weather conditions; (iii) highly-resistant materials: the material used is highly resistant, preventing degeneration (rips, tears, etc.) by abrasive contact with the surface on which the patient lies (floor of a home, pavement, stony ground, etc.).

Viscoelastic material in the padded interior of the device: the inner viscoelastic material dampens irregular movements during transfer and absorbs vibrations produced during said transfer, preventing the patient from feeling more pain and the need to use the vacuum mattress. Furthermore, it favours (without compression on the injured area and the affected limb) adaptability of the immobilisation device to the specific anatomy of the injured subject and thereby perfect immobilisation.

Radiotransparent or radiotranslucent materials: the fact that the present immobilisation device is designed and made with materials translucent to x-rays makes it easier to be able to subject the injured patient to said x-ray session without having to remove the immobiliser (SID). In other words, there will be no need to bother the patient until he is brought to the traumatologist of an emergency department.

Ease of use, polyvalency, flexibility, adaptability, and versatility: the specific immobilisation device herein described is very easy to use, where it can be used as soundly by all types of professionals and trained rescue staff with or without experience. The light weight and compact design of the device all constitute advantages for the professional who has to go to where the injured subject is. The present device can be used by all types of professional rescue teams (without the team having to have a physician), being particularly useful for rapid intervention units having different professionals working in consecutive shifts. It can be adopted by any rescue team worldwide, i.e., without any type of geographical limitation; the device lends itself to an easy production and marketing adapted to the specific requirements of different health regulations worldwide and to the requirements of different rescue units existing around the world.

The present device can also be put to use in all types of environments: urban, rural, mountain and high-lying mountain environments; in a maritime area; etc., and can be used in all types of conditions (ranging from simple falls at home or on the street to injuries in hard-to-access areas, a natural disaster area, or conflict-affected areas) and circumstances (indoor/outdoor, day or night, high or low temperatures, or adverse weather conditions, etc.). The device can be applied in a wide range of situations in combination with other specific/general immobilisation systems, where it can be used on all types of injured subjects regardless of age, build, and sizes. The device has been conceived to be used on a large majority of the population (which, according to recent statistical studies, has a waist circumference of 81 to 127 cm). The straps of the upper bands have a large size to firmly hold large-sized patients. Pregnant women can be taken care of using the device (SID) without putting the foetus or the mother in harm's way.

The advantages provided by the use of this device as will be described below are at least the following:
1. Maximum pain reduction: the use of the present device allows minimizing the pain caused as a result of the handling and movements typical of the process of rescuing the injured subject and transferring him to the hospital, improving patient care and well-being. The use of the present specific immobilisation device (SID) has a fundamental advantage with respect to the current way of evacuating, transferring, and receiving the injured subject in the hospital. The present device allows the patient to have his affected limb perfectly immobilised throughout the entire process, thereby eliminating the extra pain suffered by the patient as a result of the handling, movements, vibrations, and shifting typical of the entire rescue process. This advantage is particularly significant in those cases in which the patient cannot remain in a horizontal position once he has been restrained on the "scoop stretcher" in which he is evacuated; i.e., in those cases in which the rescue staff must tilt the stretcher and cause the patient to be in an upright position in order to go down the stairs (gradual tilting) or to use a lift for evacuation (almost vertical maximum tilting).
2. Speed and time saving: the design and features of the device make it easier for rescue to be carried out with a single specific immobilisation method which can be used throughout the entire process, i.e., during evacuation to the rescue vehicle, during transfer to the hospital centre (without being transferred to the vacuum mattress), and during handling and movement (without transfers) in said centre (including during x-ray session), and allows the time period that elapses from the moment the accident is reported to the moment the patient is brought to the traumatologist of an emergency department to be drastically reduced.
3. Lower risk of the injury worsening: in addition to eliminating pain, by means of perfect and total immobilisation of the affected limb (using its traction function, if necessary), the present immobilisation device prevents risks such as the injury worsening and even the possibility of an internal vessel rupture due to friction with the fractured bone during evacuation.
4. Savings on medical resources: Firstly, the use of the present device (SID) means that the use of medication (morphine derivatives, pain killers) used today with the described technique is unnecessary. Secondly, the use of the immobilisation device (SID) means that the use of the "vacuum mattress" for transferring the patient to the hospital is unnecessary. Thirdly, its polyvalency, the fact that a range of functions can be performed simultaneously, i.e., the fact that the immobiliser (SID) can also be used with the same efficacy, efficiency, and effectiveness for the specific immobilisation of another type of fractures (pelvic fracture, femoral fracture, knee fracture, and fracture from the proximal tibia to the knee, as well as a range of abdominal injuries) may lead to emergency services not having to carry with them other "pelvic belt"-type devices (for pelvic fractures and abdominal injuries) and/or "traction splint"-type devices (for femoral fractures and fracture from the proximal tibia to the knee) in addition to the SID. The functions of these two devices can be performed by the device (SID) herein described.
5. Savings on human resources: as discussed above, in Spain, the human team of ALS (advanced life support) units is made up of three professionals: a physician, a nurse, and a emergency medical technician (EMT), and the team of a BLS (basic life support) unit is formed by two EMTs; i.e., the BLS team is made up of only two professionals, neither of whom is authorized to medicate the injured patient. In this sense, the use of the immobilisation device (SID) allows the rescue of an injured subject in the case of a femoral neck fracture to be carried out by a BLS unit, making the intervention of an ALS unit unnecessary, i.e., making the intervention of a nurse and a physician unnecessary. The fact that the rescue process can be carried out by a BLS team, the number of which is far greater in the ambulance fleet of a city, allows the few available ALS units (as well as the limited number of physicians and nurses making up said local team) to be free to attend to more serious accidents that necessarily require the intervention of ALS units, (i.e., a physician and nurse).
6. High cost savings: the use of the described device therefore entails a significant cost savings advantage, providing, in this sense, not only significant advantages in terms of the quality of patient care, but also significant economic-financial advantages for the company providing the ambulance and/or rescue service as well. Each of the improvements and savings mentioned above (such as for example: (i) speed and subsequent time savings in each service that is performed; (ii) savings caused by the medication supplied with the current technique being made unnecessary and the need to carry different equipment to perform functions which the immobiliser (SID) can perform by itself being made redundant; (iii) savings on human resources leading to the presence of a physician for evacuating the patient, and therefore the presence of a complete ALS team, being unnecessary) entail saving the high added cost resulting from the use of the current rescue technique.

### BRIEF DESCRIPTION OF THE DRAWINGS

To complement the description of the immobilisation device at hand and for the purpose of helping to better understand the features thereof, a set of drawings of a selected preferred practical embodiment is attached as an illustrative and non-limiting integral part of said description. It must be borne in mind that identical or duplicate or equivalent or similar structures, elements, or parts appearing in one or more of the drawings are generally designated with the same natural reference number, optionally with an additional letter or additional letters to distinguish between objects or variants of similar objects, and they may not be named and/or described repeatedly. The dimensions of the components and features shown in the drawings are also chosen for the sake of convenience or clarity of presentation and are not necessarily shown to scale or in actual perspective. Finally, for the sake of convenience or clarity, some elements or structures are not shown or are shown only partially and/or with a different perspective or from different viewing points.

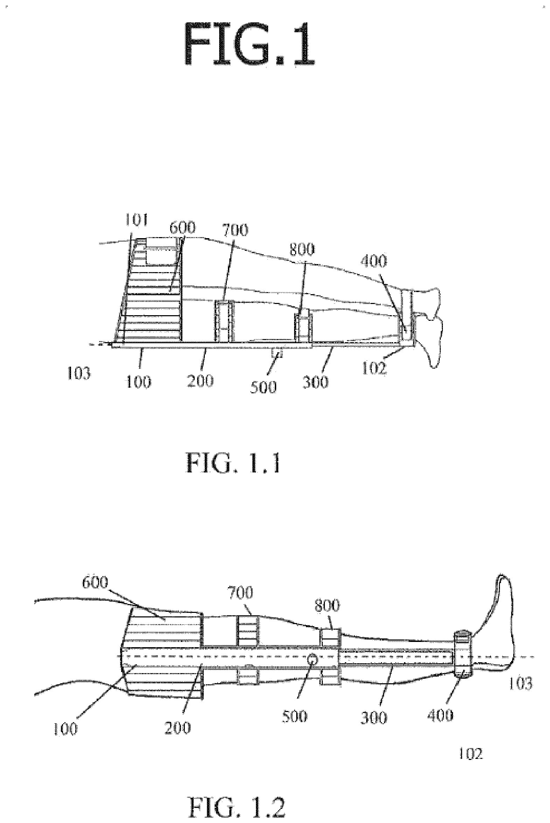

Figure 1 schematically shows the application of a preferred practical embodiment of the immobilisation device (SID) by means of two illustrations: one showing a top view of the immobilisation device (SID) duly fitted to the anatomy of the patient (Figure 1.1), and another showing a side view of the immobilisation device (SID) in the same situation (Figure 1.2).

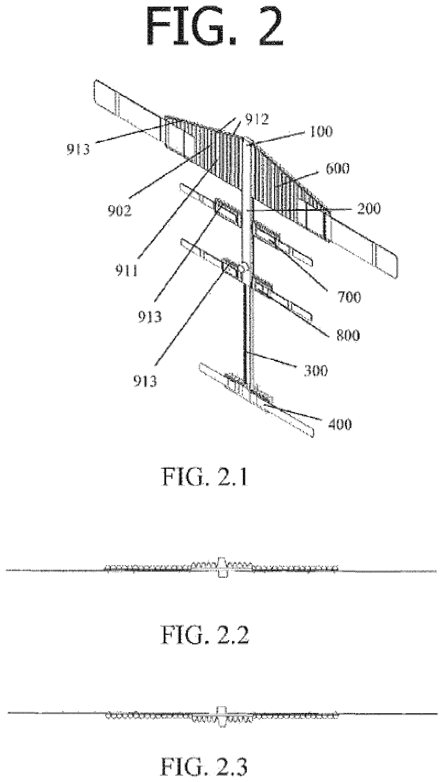

Figure 2 schematically illustrates a three-dimensional top view of a preferred practical embodiment of the immobilisation device (SID), showing the outer or non-padded face of the device (SID) (Figure 2.1). Figures 2.2 and 2.3 show a side view of the immobilisation device (SID) that is completely open (with the bands or wings completely spread out) from a top distal perspective, resting on the inner face thereof (Figure 2.3) and the same but resting on the outer face thereof (Figure 2.2).

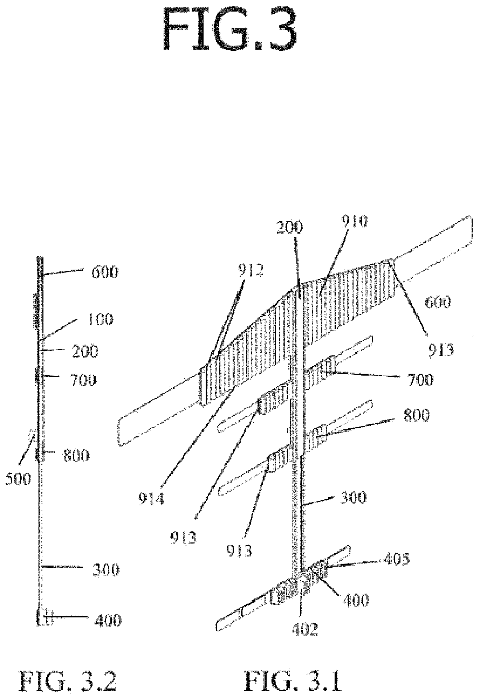

Figure 3 schematically shows a three-dimensional top view of a preferred practical embodiment of the immobilisation device (SID), showing the inner or padded face of the device (Figure 3.1). A side view of the immobilisation device (SID) can also be seen (in Figure 3.2), resting on the distal end thereof (area of the ankle band), with the telescopic central guide fully extended.

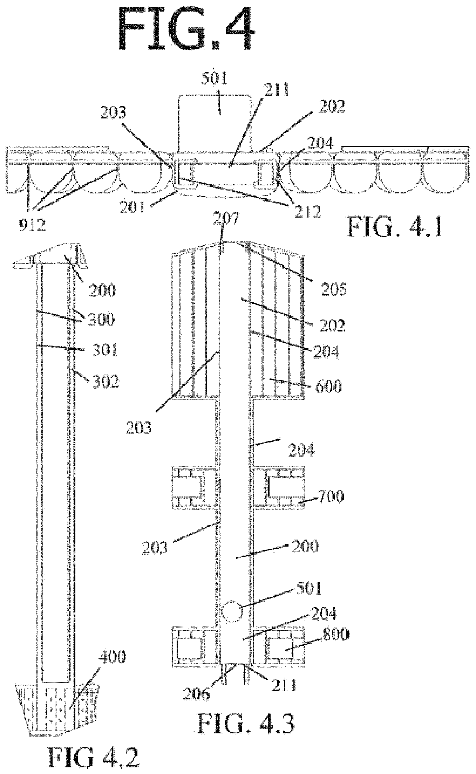

Figure 4 schematically illustrates a cross-section of the inside of the casing (Figure 4.1) of the central guide which allows seeing details of the inside thereof. Figures 4.2 and 4.3 show a top view of the movable element of the central guide, all this according to exemplary variants of the device (SID) at hand.

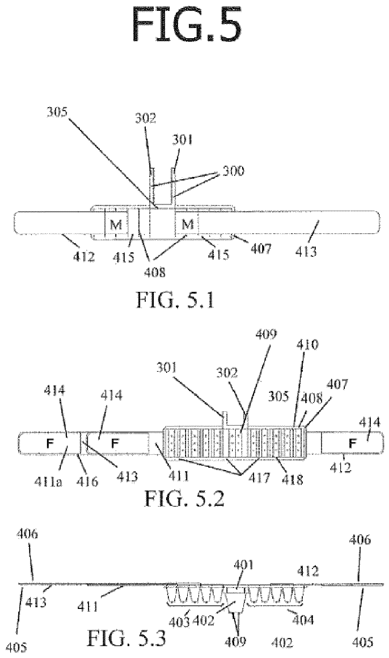

Figure 5 schematically illustrates three different views of the same element, i.e., the ankle band, of a preferred practical embodiment of the specific immobilisation device (SID). Figures 5.1 and 5.2 show the inner or padded face of the device (SID) (Figure 5.2) and the outer or non-padded face of the device (Figure 5.1). Figure 5.3 shows an inverted side view of the device (SID) from a top distal perspective.

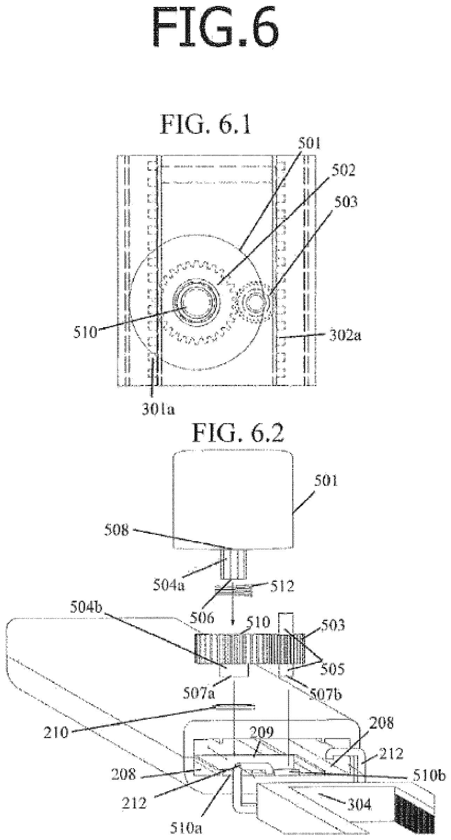

Figure 6 schematically shows a top view of the inside of the casing of the central guide (Figure 6.1) and a three-dimensional side view (Figure 6.2) in which all the elements making up the mechanism which allows the extension and retraction of the movable element of the central casing and the performance of the traction function of the affected limb are shown.

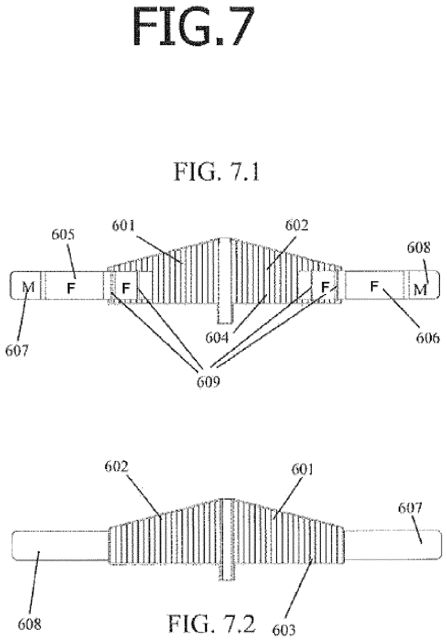

Figure 7 schematically illustrates a top view of the upper wings or bands of a preferred practical embodiment of the specific immobilisation device (SID) completely spread out or extended; the first wing or band (Figure 7.1) showing the outer or non-padded face of the device (SID) and the second wing or band (Figure 7.2) showing the inner or padded face of the device (SID).

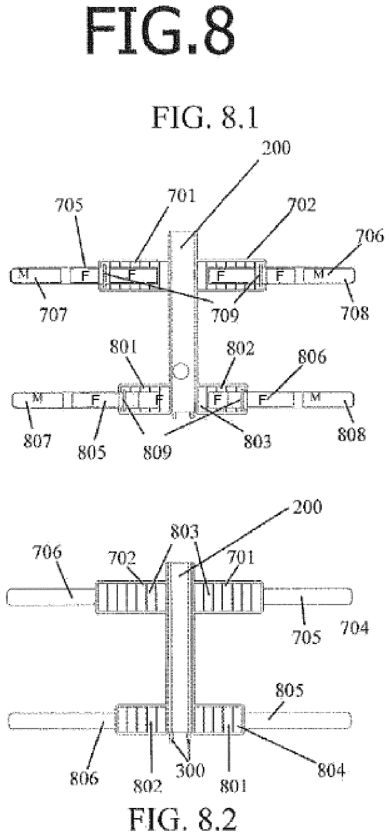

Figure 8 schematically shows a top view of the assembly formed by intermediate wings or bands and lower wings or bands of a preferred practical embodiment of the specific immobilisation device (SID). The first drawing (Figure 8.1) shows the outer or non-padded face of the device (SID), whereas the second drawing (Figure 8.2) shows the inner or padded face of the device (SID). The third drawing (Figure 8.3) shows a side view of the immobilisation device (SID) from a top distal perspective, and in which the device (SID) rests on its inner or padded face.

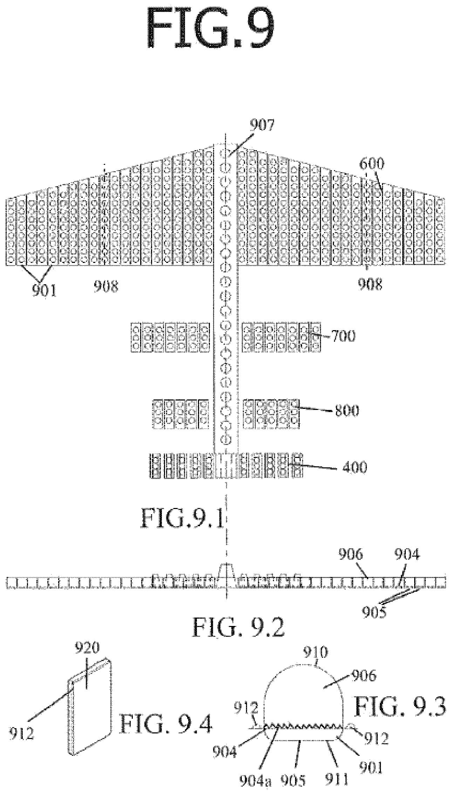

Figure 9 schematically illustrates a top view of the set of sheets forming the endoskeleton which a preferred practical embodiment of the immobilisation device (SID) has therein (Figure 9.1). Said endoskeleton is seen from a top distal perspective in Figure 9.2. Figure 9.3 shows the detail illustrated by a cross-section of one of the multiple longitudinal semi-cylindrical channels marking the various bands or wings of the device (SID), i.e., of the assembly formed by the semi-rigid sheet making up the endoskeleton and the longitudinal layer of viscoelastic material, which is adhered thereon. The last drawing (Figure 9.4) shows a three-dimensional top view of a preferred practical embodiment of the cushion that goes along with and complements the present specific immobilisation device (SID).

### DETAILED DISCLOSURE OF A PREFERRED EMBODIMENT

In view of the mentioned drawings and according to the adopted numbering, a preferred embodiment of the immobilisation device at hand, which comprises the parts and elements that are indicated and described in detail below, can be seen therein. Said description relates to one or more non-limiting examples of preferred variants of a practical embodiment of the immobilisation device (SID). This invention is not limited by the variants described or by the drawings shown, where it can be carried out to practice in different ways with different configurations, variations, and with variable dimensions. The terminology used herein must not be construed as limiting, unless otherwise specified. Likewise, the titles of the section used herein are for the sake of convenience and must not be interpreted as limiting the scope of the specific immobilisation device (SID) shown herein.

As discussed above, the immobilisation device (SID) constitutes a newly developed specific immobilisation device which allows a perfect "en bloc" immobilisation of the site of injury and the affected lower limb in cases of femoral neck fracture, the main purpose of which is to enable the efficacious, efficient, and effective, and particularly pain-free, rescue of the injured subject, all without requiring the administration of medication to the patient, and accordingly without requiring the intervention of physicians and/or nurses during the rescue process. This device can also be used with the same efficacy, efficiency, and effectiveness as a specific immobilisation device in cases of pelvic fracture, femoral fracture, knee fracture, and fracture from the proximal tibia to the knee.

The present specific immobilisation device (SID) comprises the following elements, all of them roughly shown in the attached Figure 1.1 and Figure 1.2:

### Central guide (100)

This is a central component which, like a spinal cord, performs the function of a backbone and a support for the assembly of the immobilisation device, said component being in the form of a rigid central guide having an adjustable telescopic structure (100). This structure in turn has the following elements:

### Central casing (200): Fixed rigid element

This constitutes the first of the two segments making up the mentioned central guide having a telescopic structure (100). The casing (200) represents the main support element of the central guide (100) and of the assembly of the device.

As can be seen in detail in Figure 4.1, the fixed rigid element with a hollow tubular structure of the central guide (100) referred to as the "central casing" (200) for descriptive purposes is formed by a hollow elongated tube having a rectangular section and straight edge on its outer face (202) and an edge with a smaller radius on its inner face (201). Said hollow tubular structure is manufactured in high-density polyethylene (or another material having similar characteristics/properties). Said casing is sealed at its upper end (205) and open at its lower end (206), having four faces with the two opposing faces being identical to one another (Figure 4.1). This therefore allows distinguishing two wide faces [face (201) and face (202)], one of which constitutes the inner face (201) that comes into contact with the patient, and the other of which constitutes the outer face (202) that incorporates a space so that (not directly on the casing, but rather on the material covering it in the manner that will be described below) the device (SID) has, silkscreen-printed thereon, the distinctive mark or sign chosen by the manufacturer for identifying the device when it is placed on the market; and two narrow faces [face (203) and face (204)] from which the upper bands (600), the lower bands (800), and where appropriate, the intermediate bands (700) extend, without interruption, at different heights of the central casing (200).

Cover: the entire immobilisation device (SID), i.e., both the central casing (200) and the so-called upper bands or wings (600), lower bands or wings (800), and where appropriate, intermediate bands or wings (700), are covered by respective thin polyvinyl chloride layers (or another material having similar characteristics or properties). As indicated in Figures 2.1 and 3.1, a first layer covers the padded inner face (910) of the assembly of the device (SID), which will come into contact with the patient, and another layer covers the non-padded outer face (911) of the assembly of the device (SID). These two layers would be fixed and fastened together by means of (i) a series of vertical seams (912) indicated in Figure 3.1 running longitudinally and equidistantly along the bands of the device, and (ii) by means of a border (913) consisting of a thin band of polyester textile material (or another material having similar characteristics or properties) which, as it is sewn with a seam (914), runs along the perimeter of bands (600), bands (800), and where appropriate, bands (700), and the perimeter of the casing, fixing both the inner layer (910) and the outer layer (911) and reinforcing the entire outer edge of the device (SID) (Figures 2.1 and Figures 3.1). There are fixed on the cover of the outer face of the casing (202) silicone ovals (207) which allow completing the immobilisation of the injured subject by means of fastening said subject to the spine board on which he rests "already immobilised" by means of the immobilisation device (Figure 4.3). These ovals are integrated in the casing and adhered (with industrial contact adhesive) to its structure (200). The outer face of these ovals is in direct contact with the spine board, increasing the adherence of the outer part of the assembly of the immobilisation device, enhancing the adherence of the material covering it, thereby preventing the patient from sliding on said board during evacuation, even in the cases in which he must be evacuated in a tilted or completely vertical position.

Interior of the central casing (200): the interior of the central casing houses the second segment (300) of the telescopic structure of the central guide (100) which, when extended using the gear mechanism (500) that can be seen in detail in Figures 6.1 and 6.2, enables adjusting the entire length of the central guide (100) to fit to the anatomy of the injured subject, extending said segment (300) in a controlled manner along the affected limb (Figures 1.1, 1.2, and 4.2) until aligning the "ankle band" fixed at the distal end (400) with the ankle of the injured subject.

Rails: The two solid guides (301) and (302) having a rectangular section that have been described and form the structure of the mentioned extendable segment (300) of the telescopic central guide (100) are always kept in place and aligned as a result of U-shaped rails (208) that run along the entire length of the inner side of the smaller faces (203) and (204) of the casing (see Figures 4.1, 4.2, and 6.2), and are integrated in the body of the casing (200), allowing total stability. The rails (208) have at their distal end a stop in the form of a flange (209) which helps to keep the guides within the rails at all times (see Figure 6.2) and prevents them from accidentally hitting the mechanism (500) housed therein and causing the telescopic element (300) from completely coming out of the casing in which fastening (200) is present. In other words, the guides slide along said rails (208), preventing at all times (both horizontal and vertical) shifting or movement during extension and retraction, preventing potential deformations of the tubular structure of the movable or telescopic element (300) and "clearances" which may cause said structure to not be properly fitted to the anatomy of the injured subject.

Cut-out: The lower half of the outer face (202) of the casing (200) has a cut-out (210) which enables the connection of the gear system (500) housed therein with a lever or handle in the form of an outer wheel (501) through which the aforementioned mechanism (500) is manually operated (see Figure 6.2).

Lid: As mentioned above, the upper side (205) of the casing (200) is blocked off forming a closed structure with the same physical characteristics as the rest of the structure of the casing (200). In contrast, the lower end (206) of said casing (200) is open in principle. However, this lower end (206) will be depicted as being sealed by a lid (211) [see Figure 4.1] having only two holes, reinforced by respective rectangular metal washers (212) (or washers made from another material having similar characteristics/properties) [see Figure 4.1] which prevent friction and wear, the dimensions of which are exactly the same as the perimeter of the two guides (301) and (302) forming the structure of the extendable telescopic element (300) of the guide (100). The interior of the casing (200) and its mechanism (500) are thereby protected and isolated from dirt, elements that may obstruct its operation, etc., while at the same time the fastening function of the U-shaped rails (208) described above is reinforced and the aligned extension and withdrawal thereof throughout the entire service life of the device (SID) is enabled [Figure 4.1].

Accordingly, the central casing (200), as a fixed element of the central guide (100), allows performing the following functions: (i) the function of a backbone and support, like a spinal cord, for the assembly of the device (SID), the function of a casing, housing the extendable or telescopic element, as well as the mechanism regulating same, allowing access through the structure thereof to operate it from the outside; (ii) the function of supporting and stabilizing the affected lower limb with the injured subject in a vertical, horizontal, or titled situation; (iii) the function of aligning the affected lower limb; (iv) the function of preventing the body of the patient from sliding when he is in a tilted or vertical position on the spine board during evacuation; (v) and finally, it is a key piece that enables the integral function of the device, which is to provide a perfect and specific immobilisation which allows pain to be maximally reduced during the handling and movement of the patient in a pre-hospital rescue operation (made up of the three mentioned phases: evacuation, transport, and reception) without requiring the presence of a physician and nurse, and without the administration of medication to ease the pain.

### Telescopic segment (300): rigid extendable element

As discussed above and as can be seen in Figures 1.1 and 1.2, the central guide (100) has a telescopic structure with two longitudinally successive pieces or components having the following functions: (i) the first one works as a main support and casing element (200), and (ii) the second one works as an extendable movable element (300) such that the length of the central guide (100) can be adapted to the anatomy of each patient, with the "ankle" band (400), which is secured to the distal end of the central guide (100), always being positioned (during use) at the height of the ankle of the injured subject. The extendable segment (300) of the telescopic central guide (100) is made up of (see Figures 2.1, 3.1, and 4.2) a structure formed by two rigid tubular guides [guides illustrated as (301) and (302), respectively], located parallel to one another forming a symmetrical assembly. These guides slide in unison as a single unit on the mentioned U-shaped rails (208). When they come out of the casing (200), they extend in the direction opposite said casing and remain aligned with the central casing (200) at all times, therefore enabling the mentioned first segment (200) and second segment (300) to form a rigid and stable functional whole: the so-called telescopic central guide (100) [see Figures 1.1 and 1.2].

These guides (301) and (302) have a solid tubular structure with a rectangular section, where they can be manufactured from aluminium (or another material having similar characteristics/properties). Additionally, both guides are maintained as a rigid equidistant assembly by respective tubular elements welded (preferably) in a perpendicular manner (to the guides) at both ends of the segment [upper section (304) and lower section (305)] forming a rectangular tubular structure with a closed perimeter (300). The mentioned four sections comprise the two long sections and the two short sections, where the two opposing long sections are identical to one another and the two opposing short sections are identical to one another, and they (preferably) have an identical tube section. The observer will not be able to see the two short sections given that the upper end of the extendable segment of the guide (300) is always inside the casing (200), with the stop in the form of a flange (209) mentioned above assuring that this is the case; and the lower end is embedded in what is referred to as an "ankle band" (400) for descriptive purposes, making the observer feel like the structure is only made up of an independent double tubular guide. Said structure is therefore extremely robust and compact, being resistant to deformations that may be caused by rough and unpractised use of the immobilisation device (SID).

As discussed, the extendable segment (300) of the telescopic central guide (100) provides support at its lower distal end (305) to the so-called "ankle band" (400), forming a T-shaped structure (seen clearly in Figures 2.1, 3.1, 5.1, and 5.2). Said distal end (305) of the extendable segment (300) at hand is embedded in a small hollow rectangular casing (401) made of high-density polyethylene (or another material having similar characteristics/properties) which is housed inside the "ankle band" (400) [see Figures 5.1 and 5.2]. Said casing only has space therein for said distal end (305) to fit tightly inside it. The opening existing inside said casing will be blocked off completely by the same tubular structure (305) that has been described, forming a solid block. The distal end (305) of the extendable segment (300) will be fixed with a glass fibre-reinforced polyurethane adhesive (or a similar alternative adhesive substance) [Figure 5.3].

The casing (401) is completely covered by a layer of polyvinyl chloride (or another material having similar characteristics/properties) and has two rectangular metal washers (or washers made of another material having similar characteristics/properties), being arranged around the perimeter of both guides (301) and (302), performing the function of protecting the cover material from possible friction and wear due to the intensive use of the central guide (100).

The extendable element (300) of the central guide (100) allows achieving the following functions: (i) the function of aligning the affected limb in cases of femoral neck fracture; (ii) the function of enabling the traction of the affected limb by means of using the millimetric gear mechanism provided for the use thereof in the case of femoral fracture or fracture from the proximal tibia to the knee; (iii) the function of reducing haematomas and the worsening of the injury or fracture in the preceding cases; (iv) the function of reducing pain when resetting the bone in the affected limb; and in any case (v) the function of specifically immobilising the affected limb and both lower limbs en bloc.

### Lower "ankle band" (400)

This band (400), referred to as "ankle band" for descriptive purposes (as it performs the function of fixing the central guide (100), securing the lower end (102) thereof to the ankle of the injured subject) is secured to the distal end (305) of the rigid extendable component (300) of the central guide (100) arranged perpendicular to the backbone (103) of the telescopic central guide (100), forming a T-shaped structure therewith (Figures 2.1, 3.1, 5.1, and 5.2).

The casing (401) made of high-density polyethylene (or another material having similar characteristics or properties) housed therein constitutes the base on which there is adhered (with industrial contact adhesive) a larger trapezoidal central element (402) made of high-density polyester foam (or another material having similar characteristics or properties) (Figure 5.3). As can be seen in Figure 5.3, there are arranged in a parallel manner on both sides of said larger trapezium (402) two symmetrical bands [illustrated as padded band (403) and padded band (404)], each with a series of five trapeziums with smaller dimensions (and identical material) adhered (with industrial contact adhesive) on a base made of high-density polyethylene (or another material having similar characteristics or properties) that performs the function of an endoskeleton (as in the case of the other upper band or wing (600), and intermediate band or wing (700), and lower band or wing (800) of the immobilisation device SID, as described below). The mentioned trapezoidal structures form the "padded" or inner face (910) of the "ankle band" (see Figure 3.1 and Figures 5.2 and 5.3), performing the function of going around the ankle of the affected limb, being perfectly adapted to the anatomy of the injured subject (without compression), and therefore fulfilling the purpose of allowing perfect immobilisation. It must be highlighted that the upper face of the larger central trapezium (402) on which the ankle of the injured subject rests has a curved anatomical shape to facilitate a perfect fit with respect to the limb, and accordingly perfect immobilisation.

The assembly is covered by two sheets, i.e., an inner sheet in contact with the patient and covering the padded face (405) (see Figures 3.1 and 5.2), and another outer sheet (406) (see Figures 2.1 and 5.1) made of polyvinyl chloride (or another material having similar characteristics or properties), both being sewn together (i) by means of a double perimetral seam (407), and without having [unlike in the case of the perimeter of the rest of the device (SID)] a perimetral polyethylene band in the form of a border which performs the function of reinforcing the edges of the "ankle" band (400), and (ii) by means of longitudinally arranged seams (408) separating and fixing each of the trapezoidal elements (402, 403, and 404) described above in a parallel manner (see Figures 5.1 and 5.2). As can be seen in Figures 5.2 and 5.3, on the padded inner side of the described assembly (405), and specifically on the upper face of each of the trapezoidal elements described above [on the cover (405) made of polyvinyl chloride (or another material having similar characteristics or properties)], there are adhered (with industrial contact adhesive) a series of "semicircular non-slip beads" (409) and (410). They are arranged, without limitation and for the intended purposes, in a line of three in each of the ten smaller elements (409) and in two lines of three (six in total) in the trapezoidal central element (410).

Two short straps: two straps (411) and (412) extending perpendicular to the backbone (103) of the immobilisation device (SID) in the same direction of the bands (403) and (404) are sewn on the outer face (406) of the cover layer by means of seams (415) sewn with a nylon thread (or another material having similar characteristics or properties). These straps will be manufactured from a mixed fabric of nylon and polyester (or another material having similar characteristics or properties) and incorporate a contact-type quick fastening and unfastening system, i.e., VELCRO® (414), that is fixed to the straps by means of perimetral seams (416) sewn with a nylon thread (or another material having similar characteristics or properties), said straps surrounding the ankle of the affected leg, and completing and enabling the holding of the described ankle bands (403) and (404) (see Figures 5.1 and 5.2).

Long strap: additionally and as can be seen in Figures 5.1, 5.2, and 5.3, the "ankle band" (400) has a third strap (413) that is considerably longer than the two straps (411) and (412) described above, the seam (415) sewn with a nylon thread (or another material having similar characteristics or properties) on one of the small straps (411). The long strap has the function of completing the immobilising function of the assembly of the "ankle" band (400), where it goes around and fixes both the legs of the injured subject so that his limbs are completely aligned and form a compact and stable "block". This long strap (413) also incorporates a contact-type quick fastening and unfastening system, i.e., VELCRO® (414), that is fixed to the strap by means of a perimetral seam (416) sewn with a nylon thread (or another material having similar characteristics or properties).

*/*/ Velcro®-type fasteners: both short straps (411) and (412) have a female (or smooth) fastening side (designated with the letter F in the corresponding Figure 5.2) on its inner face and a male fastening side (designated with the letter M in the corresponding Figure 5.1) is located on the outer face of the wings, such that when they are moved towards one another for anchoring thereof, the female side (of either of the two straps) is always arranged on the male side (of either of the two), which remains fixed in place. The same principle applies for the long strap (413) (which is only on one of the two sides, on the strap (411) having the female side (F) on the inner face of the strap and the male side (M) on the outer face of the same band or wing (411) from which the long strap emerges or on which the long strap is fixed.

Padded band: finally, reference is made to a small padded band (417) on which the patient's foot rests and the purpose of which is to prevent chafing. The band will be sewn forming a seam (418) with a nylon thread (or another material having similar characteristics or properties)] along the lower edge of the "ankle" band (400); it will be padded with a high-density polyester foam (or another material having similar characteristics or properties), and it will be covered with polyvinyl chloride material (or another material having similar characteristics or properties) identical to the one used for the cover of the rest of the immobilisation device (SID).

This element ("ankle band") allows performing the following functions: (i) complete fastening of the device (SID) to the ankle of the injured subject (which will be placed at a different height depending on the particular anatomy of the subject); (ii) enables perfect "en bloc" immobilisation and alignment of both limbs in conjunction with the telescopic element (300) of the guide (100), and therefore a safe and pain-free evacuation.

### Operating mechanism (500)

The central guide (100) of the SID having, as mentioned, a telescopic structure consisting of two segments (200) and (300) can be adjusted by means of an internal mechanism (500). As can be seen in Figures 6.1 and 6.2, the central casing (200) houses therein a simple straight tooth gear mechanism (500) formed by an assembly of two parallel-axis gears or wheels: (i) a larger gear (502) performing the function of a "gear ring" or drive gear and making up the element on which thrust or force is manually exerted from outside the device (SID) through a "handle" or "wheel" (501) with which it is connected, and (ii) another smaller gear (503) performing the function of an "integral pinion" with respect to the preceding larger gear. They are both located in a horizontal position, meshing with one another on one hand, and in turn meshing with the "indentations" of the inner faces (301a) and (302a) of the respective guides (301) and (302) (see Figure 6.1) on the other hand. The assembly, i.e., the two gears, the larger gear (502) and smaller gear (503), the two sections of the shaft of the larger gear which are illustrated as shaft (504a) and shaft (504b), and the shaft of the smaller gear (505), are designed to be manufactured from aluminium (or another material having similar characteristics or properties).

The upper section of the shaft of the larger gear or gear ring (504a) is fixed to the "handle" in the form of a "wheel" (501). This wheel is manufactured from high-density polyethylene (or another material having similar characteristics or properties) and enables manually operating the internal gear mechanism. It must be pointed out that said "handle" is covered with a rough anti-slip material (or another material having similar characteristics or properties) to make it easier to grip same in all types of conditions. When said upper section of the shaft of the larger gear or gear ring is manually operated (by pulling it outward), the lower end of the shaft (506) comes out of the notch (510) provided for that purpose at the central point of the "larger gear" or "gear ring". When said handle (501) is no longer operated, said lower end of the shaft (506) again rests in said notch (510). The lower section of the shaft (507a) of the larger gear and the lower portion (507b) of the shaft of the smaller gear remain in place as a result of the notches (510a) and (510b) with a high border (see Figure 6.2) present on the inner face of the base (201) of the central casing (200). There is located on the larger gear a spring (512) which allows operating the mechanism causing the movable or extendable segment of the guide (300) to be held and released. The two ends of said spring (512), i.e., the upper end [in contact with the "handle" or "wheel" (501)] and the lower end [on the surface of the larger gear] are fixed, said spring surrounding the upper piece of the shaft of the gear (504a). When said spring is in standby [i.e., when the lever or handle (501) outside the casing (200) is not operated], the mechanism works by contributing to assuring that the guides (301) and (302) remain in place and do not move. In contrast, when the spring is in the elongated state, i.e., when it is elongated as a result of the manual operation of the lever (501), the mechanism works by releasing the upper section of shaft (504a) from the notch of the larger gear (510), allowing both gears (the larger gear [502] and the smaller gear [503]) to rotate about their shafts [(504b) and (505)]. The guides [(301) and (302)] can thereby be extended as much as needed in a single movement when the professional rescuer manually pulls on them, taking them out of the central casing (200) in which they are located in standby.

There is a need to highlight that although the millimetric "bite marks" marking almost the entire inner face of both guides (301a) and (302a) are identical (in the sense that they have the same dimensions), they are not placed in a symmetrical manner, one in front of another. In other words, in order to allow both guides [(301) and (302)] to be extended or withdrawn in unison, they are "synchronized", in a manner of speaking, in an asymmetrical position, one with respect to the other. Likewise, it should be borne in mind that the "bite marks" depicted in Figure 6.1 have been amplified for descriptive purposes, therefore they intentionally do not coincide with the dimensions of the teeth of the gears that must be inserted therein.

In this manner, through the described manual operation [pulling on the lever or handle (501)], the internal mechanism of the immobilisation device (SID) performs three functions: (i) on one hand, it keeps the extendable telescopic segment (300) of the guide (100) held inside the casing (200) when it is withdrawn or in standby; (ii) on the other hand, it allows extending the telescopic or extendable segment (300) of the guide (100), aligning the affected limb and holding it securely in place once the "ankle band" (400) has been placed at the level of the ankle of the injured subject; (iii) finally, in cases of femoral fracture and fracture from the proximal tibia to the knee, the millimetric gear mechanism (500) described above is designed to allow the mentioned telescopic segment (300) of the guide (100) to be extended "millimetre by millimetre" by means of rotating the wheel, which allows a delicate and precise traction of the affected lower limb.

Upper bands or wings (600), intermediate bands or wings (700), and lower bands or wings (800)
Once again, these elements of the immobilisation device (SID) are referred to as "bands" or "wings" only for mere descriptive purposes and without having any limiting character whatsoever. As can be seen particularly in Figures 2.1 and 3.1, the guide or central casing (200) has a total of six sections connected or fastened on both sides of the casing.

The bands or wings emerge from both sides of the segment of the central guide (100) referred to as "central casing" (200) and extend in a perpendicular line in the direction opposite the vertical backbone (103) of the central guide (100). The aforementioned bands or wings provide the device (SID) with both vertical rigidity and horizontal flexibility as a result of the unique longitudinal equidistant arrangement of a series of sheets which together form a type of semi-rigid "endoskeleton" (900) shown in Figure 9.1. There are typically three types of bands: (i) a set of two upper bands (600) that will be placed at the height of the pelvic area of the patient (Figures 7.1. and 7.2) symmetrically arranged on both sides of the guide [band (601) and band (602)], having a "non-padded" face (604) or outer face and a "padded" face (603) forming the inner face which will come into contact with the body of the injured subject. As can be specifically seen in Figures 7.1 and 7.2, the function of the bands is extended in the form of straps [strap (605) and strap (606)] that are fitted around the injured subject. The second set of bands are (ii) an (optional) set of two intermediate bands (700) that will be placed, where appropriate, at the height of the femoral area of the patient (Figures 8.1. and 8.2) symmetrically arranged on both sides of the guide [band (701) and band (702)], also having a "padded" face (703) which forms the inner face of the bands and will come into contact with the body of the injured subject, and a "non-padded" face (704) or outer face. As can be specifically seen in Figures 8.1 and 8.2, the function of the bands is extended in the form of straps [strap (705) and strap (706)] that are fitted around the injured subject. The third set of bands are (iii) a set of two lower bands (800) that will be placed at the height of the tibial area of the patient (Figures 8.1. and 8.2) symmetrically arranged on both sides of the guide [band (801) and band (802)], having, like the other bands, a padded inner face (803) and a "non-padded" outer face (804). As can be specifically seen in Figures 8.1 and 8.2, the function of the bands is extended in the form of straps [strap (805) and strap (806)] that are fitted around the body of the injured subject.

### "Endoskeleton" made of cut-out sheets (900)

Each of the side bands or sections extending on each side of the guide (601) and (602), (701) and (702), as well as (801) and (802) has an endoskeleton (900) formed by a series of rigid independent sheets (901) that run parallel to the central casing (200), along the entire interior of the device, in a continuous and longitudinal manner (see Figures 9.1, 9.2, and 9.3). Said sheets are arranged in a parallel and equidistant manner (to allow for a space in which the longitudinal seams (912) fixing the inner layer (910) and the outer layer (911) of the cover of the immobilisation device will be housed, as described above), which sheets having a straight or rectangular tubular structure with two smaller sides (902) and (903) forming the thickness of the sheet and two larger sides, one of which having the particularity having a flat "inner" face (904) [the face on which the viscoelastic material (906) will be fixed] and a curved "outer" face (905) [see Figure 9.3]. This allows the aforementioned longitudinal seams (912) to be protected from brushing against the ground [or any surface on the injured subject lies] when the device must be slid under the body of the injured subject during use [also seen in detail in Figure 4.1].

This type of inner longitudinal "infrastructure" is formed by sheets (901) made of rigid high-density polyethylene (or another material having similar characteristics or properties) with cut-outs (907) [Figure 9.1] and have a different length depending on the section (600), where appropriate (700), (800), or (400) in which they are placed. In any case, said sheets do not extend all the way to the edge of the device (SID) to allow attaching and sewing both layers [inner layer (910) and outer layer (911)] together along the perimeter, forming the border (913) described above (see Figures 2.1 and 3.1).

The aforementioned cut-outs (907) have a variable configuration, the central point of which coincides with the imaginary longitudinal axis (908) of each of the sheets (901) at hand [see Figure 9.1]. The purpose of the cut-outs is to reduce the weight of the assembly, making it more manageable, and to use the smallest amount of material possible, all this obviously without compromising the characteristics of said material and its purpose.

Each of the sheets (901) has adhered (with industrial contact adhesive) [depicted in Figure 9.3 as (904a)] to the surface of its upper or inner face (904) a longitudinal "rectangular" layer of viscoelastic material (906) covering the entire surface thereof. The assembly of said sheets and viscoelastic material forms the inner or padded face (910) that will come into contact with the body of the injured subject and allows the device to perfectly adapt to the unique anatomy of each patient (without compression) (see Figures 3.1, 9.2, and 9.3).

Function: The described endoskeleton (900) allows simultaneously combining the functions of (i) providing vertical rigidity to the assembly, extending the patient stabilization and alignment function fundamentally carried out by the central guide (100), and (ii) enhancing the flexibility and horizontal anatomical adaptation function of the bands or wings of the immobilisation device (SID) (or parallel symmetrical sections) with respect to the body of the patient. Combining both functions described above is what ultimately allows the pain-free handling, evacuation, and transfer of the patient without any medicinal products and without the intervention of any professional practitioner in pre-hospital stage of the patient's care.

Covering or cover of the device (910) and (911): As can be seen mainly in Figures 2.1 and 3.1, the immobilisation device (SID) is covered with two layers, one referred to as an "inner" layer (910) for descriptive purposes and another referred to as an "outer" layer (911) for the same purposes, arranged such that they cover the entire device [such as the central casing (200), and the upper bands (600), intermediate bands (700) where appropriate, and lower bands (800), for example; and also the "ankle band" (400)]. The material or fabric used for covering the entire device (SID) are two sheets of polyvinyl chloride (or another material having similar characteristics or properties) [for example and without limitation, each one being 1 mm thick].

Longitudinal multi-tunnel (semi-cylindrical channel) system (914): The longitudinal assembly of the sheet of rigid high-density polyethylene (901) and the strip or layer of superimposed viscoelastic material (906) is covered by respective layers of polyvinyl chloride [on its outer or non-padded face (911) and the inner or padded face (910)], being "perfectly fixed" in a equidistant manner as a result of the parallel longitudinal seams (912) attaching or fixing both sheets or inner cover layer (910) and outer cover layer (911) separating same, creating "in an alternating manner" a design consisting of padded semi-cylindrical channels (914) containing the assembly of the sheet (901) and viscoelastic material (906) and longitudinal notches in the form of a seam (912) parallel to the preceding ones [see Figures 9.3, 4.1, and 3.1].

The alternating arrangement of semi-cylindrical channels (914) [with the sheet (901) and the viscoelastic material (906) therein] and vertical longitudinal notches (seams) (912) gives the inner face of the immobilisation device (SID) a "padded appearance" [see Figures 3.1 and 4.1]. As discussed above, said padded face (910) has been designed to come into contact with the body of the patient and to allow the complete adaptation (without compression) thereof to the unique anatomy of the injured subject, allowing the complete immobilisation of the patient throughout the entire rescue process.

Outer face of the device (911): In contrast and as can be seen in Figure 2.1, the outer face (911) of the immobilisation device (SID) only has the outer face of the same seams (912) described above, sewn longitudinally/parallel to one another at equidistant intervals, but without having padding which prevents the liner from contacting the inner sheet. The fact that the outer portion (905) of the sheets (901) forming the endoskeleton has a certain curvature (see Figure 9.3) allows the mentioned longitudinal seams (912) not to come into direct contact with the surface on which the injured subject lies, preventing friction that will damage the seams (912) when the device is slid under the injured subject.

Function: The bands (600), (800), and where appropriate (700) perform two simultaneous functions: (i) they extend from the central casing (200) and provide the necessary longitudinal or vertical rigidity as a result of the assembly of parallel equidistant sheets (901) forming the endoskeleton (900) thereof; (ii) said bands in turn provide the necessary flexibility and horizontal adaptability to go around the body of the injured subject, moulding itself to the unique and specific anatomical contour of the individual at hand (without compression).

### Borders of the bands of the device (913)

The assembly formed by the central casing (200) of the guide (100) and the described sections, i.e., the set of wings/bands (600), where appropriate the set of wings/bands (700), and the set of wings/bands (800), has a band or border (913) running uninterrupted along the perimeter thereof, fixing the two layers of polyvinyl chloride (or another material having similar characteristics or properties) forming the cover of the entire device, i.e., of the padded face (910) and of the non-padded face (911) [Figures 2.1 and 3.1]. Said perimetral band or border is covered with or made of polyester textile material (or another material having similar characteristics or properties), being sewn as reinforcement, forming a reinforced perimetral seam [double stitching] with a nylon thread (or another material having similar characteristics or properties). The function thereof is to fix said covering and reinforce the perimetral area of the assembly of the immobilisation device (SID),

### Fastening straps with anchoring in the form of Velcro (or the like)

The six mentioned sections, i.e., the upper sections (601) and (602) [Figures 7.1 and 7.2], where appropriate the intermediate sections (701) and (702), and the lower sections (801) and (802) [Figures 8.1 and 8.2], have at their ends straps [designated in the preceding drawings as (605/606), (705/706), and (805/806) that follow the direction of the bands, i.e., perpendicular to the backbone (103) of the central casing (200) [Figure 1.1], extending the reach of the sections around the body of the injured subject. As can be seen in Figures 7.1 and 7.2, the straps of the upper bands (605/606) have a size considerably larger than the straps of the intermediate bands (705/706) and lower bands (805/806) [visible in Figures 8.1 and 8.2]. The straps are made of polyester textile material (or another material having similar characteristics or properties) and are sewn with a nylon thread (or another material having similar characteristics or properties) to the outer layer covering the bands or wings of the immobilisation device. Unlike the longitudinal seams that mark the assembly of the device (912) and are visible on both faces [the inner face (910) and the outer face (911)], this seam is only on the outer side of the device (SID) and does not go into the inner face given the existence of the longitudinal sheets (901) forming the endoskeleton [see Figures 7.1 and 8.1]. The mentioned straps reinforce and complement the functions of completely fastening and immobilising the injured subject provided by the bands or wings (600), (800), and where appropriate (700) of the device (SID).

In terms of the anchoring system, the immobilisation device (SID) has a Velcro®-type quick fastening and unfastening system. The straps of the bands or wings of the device (600), where appropriate (700), and (800) always have "the Velcro® side" of the fastener on the outer face (604), (704), and (804) of the corresponding strap. Both straps in each of the bands or wings, such as (605/606), (705/706), and (805/806), for example, have two female sections in the inner portion of the strap followed by a male section at the end farthest away therefrom (designated with the letter F (for female) and M (for male), respectively, in Figures 7.1 and 8.1). The existence of the two consecutive female sections (Figures 7.1 and 8.1) along the outer face of the two straps (605/606), (705/706), and (805/806) in each of the wings or bands is so that the device can be adjusted to fit the particular anatomy of the patient. If the circumference of the abdominal area of the injured subject is larger, anchoring will be performed at a point closer to the distal end of the band, the fastener being anchored, in contrast, at a point closer to the backbone of the device.

The described fastening and unfastening system has an additional element, i.e., the sliding rings or arcs [designated as (609) [Figure 7.1], (709) and (809) [Figure 8.1], respectively] allowing for insertion of the strap through the opening thereof and for a 180 degree change in direction of "travel", being secured to the corresponding Velcro strip located on the opposite face of the strap (described above). The function of said elements is to limit the force and the subsequent compression exerted on the injured limb and the rest of the anatomy of the injured subject during the process in which the device is adjusted to fit him so that he is completely and properly immobilised to be evacuated and transferred to the hospital centre.

### 3. Complementary elements. Cushions

The "cushion" (920) depicted in Figure 9.4 has a rectangular shape where it is completely filled with a viscoelastic material (906) without any type of internal structure that performs the function of an endoskeleton. Said cushion is uniformly covered by a layer of the same material as the rest of the immobilisation device (SID), i.e., a layer of polyvinyl chloride, being fixed with standard seams (912) sewn with nylon (or another material having similar characteristics or properties) at the edges. As mentioned, the cushion is highly flexible and mouldable (can be used in a vertical and horizontal position where it can also be bent vertically or horizontally in half along the axis thereof to increase or double its volume); it can be used in different ways in the hypothetical case in which the particular anatomy of the injured subject gives rise to the existence of anatomical gaps at the height of the hip that must be filled to allow the complete immobilisation of the patient and to thereby prevent pain caused, for example, by vibrations during transfer.

### METHOD OF USING THE SID

The basic phases of a method of using the present device are described below, said method comprising the following steps:
- The emergency team (which is made up in Spain, by law, of two or three professionals) or rescue team goes to where the injured subject is located, regardless of whether he is on a public street, inside a building, in a rural area, etc.
- The emergency team or rescue team performs a quick assessment of the injured subject to establish the extent of his injury/injuries. In the event that the injured subject shows signs and symptoms suggesting a femoral neck fracture injury, the emergency professional (paramedic, physician, nurse, or emergency medical technician) or rescue professional will use this new immobilisation device (SID) in the manner described in detail below.
- The immobilisation device (SID) at hand will be placed, open or extended, resting on its outer or non-padded face, on a spine board, parallel to the injured subject at the height of his hip.
- One of the health professionals or rescuers will slide the immobilisation device (SID) under the injured subject while his teammate holds the patient in the lateral decubitus position, with the affected limb held in place.
- So while one of the professionals places and holds the patient in the lateral decubitus position and the other holds the affected lower limb, they will both proceed to slide the spine board along with the immobilisation device arranged thereon under the patient at the suitable height together and at the same time using their free hands (i.e., this action will be performed depending on the height of the site of the injury or fracture).
- Once the patient is moved back to the supine decubitus position, the professional assures that the patient is suitably located on the immobilisation device (SID).
- For the purpose of assuring a suitable venous return, rescuers will proceed to adjust the fastening straps from the bottom up, first adjusting the straps of the lower bands or wings (at the tibial level), then the straps of the intermediate bands or wings (at the femoral level) if they are included in the configuration of the specific immobiliser (SID), and finally the straps of the upper bands (at the hip and/or pelvic level).
- In the exceptional case where the anatomy of the injured subject so requires, the emergency staff or rescuer will place during the preceding process cushions which are provided and which the staff or rescuer believes are necessary in order to fill the anatomical gaps left by the particular anatomy of the injured subject, thereby assuring perfect immobilisation of the patient.
- Once the patient has been immobilised at the indicated height, the telescopic or movable section of the guide is placed and adjusted with respect to the height of the ankle of the patient. The guide will be extended depending on the length of the affected limb of the patient, so that the "ankle band" and its straps are aligned parallel to the ankle (or ankles) of the injured subject.
- The straps of the ankle of the affected limb are then adjusted.
- Traction: In the case of a hip fracture, a femoral fracture, and a fracture from the proximal tibia to the knee, traction is applied (downward or upward, depending on the case) to the affected lower limb with the guide to align the fracture, thereby minimizing pain and compensating for the lack of medication. In the case of a pelvic or knee fracture, traction would not be necessary.
- Distal pulse: Once traction is applied to the limb, the distal pulse is taken to check for the existence of blood flow and thereby distal tissue oxygenation.
- For the patient to form "a block" making his evacuation easier, the second strap of the device in the "ankle band" is adjusted. The strap is long enough to go around both extremities, the one affected by the injury and the one not affected, forming a "block" which completely stabilizes and immobilises the extremities "en bloc", minimizing movements, vibrations, and accordingly pain.
- Finally, the patient is fastened to the spine board on which he rests already immobilised using the provided fastening straps, and he is then evacuated to the vehicle used in the corresponding rescue service or operation.
- In contrast with the current technique described above, the use of the immobiliser (SID) allows the patient to be transferred directly to the hospital, without having to be moved to the vacuum mattress, i.e., the use of the immobiliser (SID) allows the patient to be transferred to the hospital centre using the same resources [spine board and immobiliser (SID)] with which he has been evacuated.
- The type of material used in the design and manufacture of the immobilisation device (SID) allows subjecting the patient to x-rays upon reaching the hospital without having to remove the immobilisation (under normal circumstances, i.e., using the current technique, the patient must first be moved from the vacuum mattress to a stretcher or bed). The patient is finally transferred from the x-ray department to be cared for by the traumatologist of an emergency department without any need to remove the immobiliser (SID) on which the patient is placed during evacuation and without any need to repeatedly move the patient. This results in an excellent quality of patient care throughout the entire process, i.e., patient handling and movement at the scene of the accident, evacuation and transfer, reception, and initial hospital care.

## Claims

1. Rescue, pre-hospital medical emergency, and a patient transport device which is in the form of a specific immobilisation device (SID) wherein it allows complete en bloc immobilisation of a pelvic area and an affected lower limb of an injured subject in cases in which a femoral neck fracture is suspected, and the device comprises:
(i) a rigid guide having a telescopic structure (100) which allows stabilising the pelvic area and perfectly aligning the affected lower limb, and having at its lower distal end a T-shaped ankle band (400) completing said immobilising function of the assembly;
(ii) a system for securing comprising a single padded anatomical band or a wing or two sets of padded anatomical bands or wings (600) and (800) wherein each of said padded anatomical bands or wings comprises a longitudinal multi-tunnel system formed by parallel longitudinal seams (912) containing a viscoelastic layer on a rigid laminar endoskeleton that runs parallel to the limb to be immobilised, confers vertical rigidity and horizontal flexibility, and allows fixing the telescopic rigid guide (100) to the body of the injured subject, encircling it, and completing the immobilisation of the pelvic area with both extremities en bloc;
(iii) a series of straps (605)/(606) and (805)/(806) with quick fastening (607)/(608) and (807)/(808) which complete the function of the bands or wings (601)/(602) and (801)/(802) into which they are inserted, allowing both opposite ends to remain duly adjusted and connected;
(iv) an internal parallel-axis, straight tooth gear mechanism (500) formed by a larger gear (502) manually exerted from outside the device (SID) through an outer handle (501) and a smaller gear (503), which gears mesh with one another and in turn with millimetric indentations of the inner side of the guides (301a/302a) facilitating, by means of manual operation of pulling on and releasing the outer handle (501), in cases of the femoral neck fracture, a mechanical extension and retraction of a telescopic or mobile element of a guide (300) and a subsequent adaptation of the ankle band (400) of the device (SID) to a specific anatomy of each injured subject.

2. Specific immobilisation device (SID) according to claim 1, **characterised by** enabling the use of the internal gear mechanism (500) by means of manual rotary operation, in one direction or another, of the outer handle (501), allowing the millimetric extension and retraction of the telescopic element of the guide (300) and the subsequent millimetric mechanical traction of the affected lower limb in cases of femoral fracture and fracture from a proximal tibia to a knee.

3. Specific immobilisation device (SID) according to any of claims 1 or 2, further **characterised in that** it has a set of central wings or bands (700) reinforcing the function of the other two sets of upper bands or wings (600) and lower bands or wings (800).

## Patentansprüche

1. Rettungsvorrichtung für einen präklinischen medizinischen Notfall und für den Transport von Patienten, welche in Form einer spezifischen Immobilisierungsvorrichtung (SID) ist, wobei sie die vollständige En-Bloc-Immobilisierung eines Beckenbereichs und einer betroffenen unteren Gliedmaße eines verletzten Individuums erlaubt, in Fällen, in welchen einen Oberschenkelhalsbruch vermutet wird, und wobei die Vorrichtung Folgendes umfasst:
(i) eine starre Führung aufweisend eine teleskopische Struktur (100), welche die Stabilisierung des Beckenbereichs und die perfekte Ausrichtung der betroffenen unteren Gliedmaße erlaubt, und welche an dessen unteren distalen Enden ein T-förmiges Knöchelband (400) aufweist, welches die genannte Immobilisierungsfunktion der Baugruppe vervollständigt;
(ii) ein Befestigungssystem, welches ein einziges gepolstertes anatomisches Band oder einen Flügel oder zwei Sätzen von gepolsterten anatomischen Bändern oder Flügeln (600) und (800) umfasst, wobei jedes/jeder der genannten gepolsterten anatomischen Bänder oder Flügel ein längliches mehrfaches Tunnelsystem gebildet aus parallelen Längsnähten (912) umfasst, welches eine viskoelastische Schicht auf einem starren flächenförmigen Endoskelett enthält, welche parallel zur zu immobilisierenden Gliedmaße verläuft, vertikale Starrheit und horizontale Flexibilität verleiht und die Fixierung der teleskopischen starren Führung (100) am Körper des verletzten Individuums erlaubt, indem er umgeben wird, und die En-Bloc-Immobilisierung des Beckenbereichs mit beiden Extremitäten vervollständigt;
(iii) eine Reihe von Gurten (605)/(606) und (805)/(806) mit Schnellbefestigung (607)/(608) und (807)/(808), welche die Funktion der Bänder oder Flügel (601)/(602) und (801)/(802), in welche sie eingeführt werden, vervollständigen, sodass es erlaubt wird, dass beide gegenüberliegenden Enden ordnungsgemäß eingestellt und verbunden bleiben;
(iv) einen inneren Stirnradgetriebemechanismus mit geraden Zähnen (500) gebildet aus einem größeren Getriebe (502), welches von außerhalb der Vorrichtung (SID) über einen Außengriff (501) manuell betätig wird, und einem kleineren Getriebe (503), welche Getriebe ineinandergreifen und wiederum mit millimetrischen Einkerbungen der inneren Seite der Führungen (301a/302a), sodass sie, mittels manueller Betätigung durch das Ziehen und Loslassen des Außengriffs (501), in Fällen des Oberschenkelhalsbruchs, eine mechanische Erstreckung und einen mechanischen Rückzug eines teleskopischen oder beweglichen Elements einer Führung (300) und die nachträgliche Anpassung des Knöchelbands (400) der Vorrichtung (SID) an eine spezifische Anatomie jedes verletzten Individuums erleichtern.

2. Spezifische Immobilisierungsvorrichtung (SID) nach Anspruch 1, **dadurch gekennzeichnet, dass** sie die Verwendung des inneren Getriebemechanismus (500) mittels manuellen Drehbetriebs, in einer Richtung oder der anderen, des Außengriffes (501) ermöglicht, sodass die millimetergenaue Erstreckung und der millimetergenaue Rückzug des teleskopischen Elements der Führung (300) und der nachträgliche millimetergenaue mechanische Zug der betroffenen unteren Gliedmaße in Fällen eines Oberschenkelbruchs und eines Bruchs von einer proximalen Tibia bis zu einem Knie erlaubt wird.

3. Spezifische Immobilisierungsvorrichtung (SID) nach einem der Ansprüche 1 oder 2, **dadurch gekennzeichnet, dass** sie zusätzlich einen Satz von mittigen Flügeln oder Bändern (700) aufweist, welche die Funktion der anderen zwei Sätze von oberen Bändern oder Flügeln (600) und unteren Bändern oder Flügeln (800) verstärken.

## Revendications

1. Dispositif de sauvetage, d'urgence médicale pré-hospitalière et de transport de patient se présentant sous la forme d'un dispositif d'immobilisation spécifique (DIE) permettant l'immobilisation complète en bloc d'une zone pelvienne et d'un membre inférieur atteinte d'un sujet blessé dans des cas dans lesquels une fracture du col du fémur est suspectée, et le dispositif comprend :
(i) un guide rigide ayant une structure télescopique (100) qui permet de stabiliser la région pelvienne et d'aligner parfaitement le membre inférieur atteint et ayant à son extrémité inférieure distale une bande de cheville en forme de T (400) complétant ladite fonction d'immobilisation de l'ensemble.
(ii) un système de fixation comprenant une seule bande anatomique matelassée ou une aile ou deux ensembles de bandes ou ailes anatomiques matelassées (600) et (800) dans lequel chacune desdites bandes ou ailes anatomiques matelassées comprend un système multi-tunnel longitudinal formé de coutures longitudinales parallèles (912) contenant une couche viscoélastique sur un endosquelette laminaire rigide qui s'étend parallèlement au membre à immobiliser, confère une rigidité verticale et une flexibilité horizontale et permet la fixation du guide rigide télescopique (100) au corps du sujet blessé, l'entourant et achevant l'immobilisation de la région pelvienne avec les deux membres en bloc ;
(iii) une série de sangles (605)/(606) et (805)/(806) à fixation rapide (607)/(608) et (807)/(808) complétant la fonction des bandes ou des ailes (601)/(602) et (801)/(802) dans lesquelles elles sont insérées, permettant ainsi aux deux extrémités opposées de rester dûment ajustées et connectées ;
(iv) un mécanisme d'engrenage à dents droits à axe parallèle interne (500) formé d'un engrenage plus grand (502) exercé manuellement de l'extérieur du dispositif (DIE) à travers une poignée extérieure (501) et un engrenage plus petit (503), les engrenages étant en prise entre eux e à son tour avec des empreintes millimétriques de la face interne des guides (301a/302a) facilitant, par une opération manuelle de tirer et de relâcher la poignée externe (501), dans des cas d'une fracture du col fémoral, une extension et rétraction mécanique d'un élément télescopique ou mobile d'un guide (300) et une adaptation ultérieure de la bande de cheville (400) du dispositif (DIE) à une anatomie spécifique de chaque sujet blessé.

2. Dispositif d'immobilisation spécifique (DIE) selon la revendication 1, **caractérisé en ce qu'**il permet l'utilisation du mécanisme d'engrenage (500) par le biais d'une opération de rotation manuelle, dans un sens ou dans l'autre, de la poignée extérieure (501), permettant l'extension et la rétraction millimétriques de l'élément télescopique du guide (300) et la traction mécanique millimétrique ultérieure du membre inférieur atteint dans des cas de fracture du fémur ou d'une fracture du tibia proximal au genou.

3. Dispositif d'immobilisation spécifique (DIE) selon l'une quelconque des revendications 1 ou 2, **caractérisé en outre en ce qu'**il comporte un ensemble d'ailes ou de bandes centrales (700) renforçant la fonction des deux autres ensembles de bandes ou d'ailes supérieures (600) et de bandes ou d'ailes inférieures (800).
